# EUROPÄISCHE PATENTANMELDUNG

(11) **EP 1 891 890 A1**
(43) Veröffentlichungstag der Anmeldung: **27.02.2008**
(21) Anmeldenummer: 07112716.1
(22) Anmeldetag: 18.07.2007
(51) Int. Cl.: A61B 3/12

(54) **Optisches Gerät, Verwendung eines erfindungsgemäßen optischen Gerätes sowie Verfahren zum Blocken von Lichtreflexen im Beobachtungsstrahlengang eines optischen Gerätes**

(30) Priorität: 21.08.2006 DE 102006039137
(71) Anmelder: Carl Zeiss Surgical GmbH, 73447 Oberkochen (DE)
(72) Erfinder: Obrebski, Dr., Andreas, 40489, Düsseldorf (DE)
(74) Vertreter: Müller, Thomas

(57) **Zusammenfassung**

Die Erfindung betrifft ein optisches Gerät zur Untersuchung eines Auges, mit mindestens einem dem Auge zugewandt angeordneten Objektiv, mindestens einer Beleuchtungsquelle zur Beleuchtung des Auges, einem bzw. einer zwischen dem Objektiv und dem zu untersuchenden Auge angeordneten optischen Element und/oder optischen Anordnung, welches bzw. welche ein Zwischenbild der untersuchten Ebene im Augeninneren erzeugt, die mit dem Objektiv betrachtet wird, wobei in dem Beobachtungsstrahlengang des optischen Gerätes zumindest ein ortsaufgelöstes Dämpfungselement angeordnet ist, welches in mindestens zwei Transparenzzustände ortsaufgelöst schaltbar ist. Ferner betrifft die Erfindung ein erfahren zum Blocken von Lichtreflexen im Beobachtungsstrahlengang eines optischen Gerätes sowie die Verwendung eines derartigen optischen Gerätes zur Untersuchung eines Auges

## Beschreibung

Die vorliegende Erfindung betrifft ein optisches Gerät, insbesondere ein Ophthalmoskop, zur Betrachtung eines Auges, insbesondere des Hintergrundes eines Auges. Ferner betrifft die Erfindung ein Verfahren zum Blocken von Lichtreflexen im Beobachtungsstrahlengang eines optischen Gerätes sowie die Verwendung eines erfindungsgemäßen optischen Gerätes.

Die Ophthalmoskopie bzw. Augenspiegelung oder auch Funduskopie dient der Beurteilung von krankheitsbedingten Veränderungen des einsehbaren Teiles eines Auges. Bei der Augenuntersuchung blickt der Betrachter mittels eines optischen Gerätes mit einem optischen Element, insbesondere einer Linse, durch die Pupille des Auges hindurch in das Innere des Auges. Hierzu muss das Auge mit einer Lichtquelle erhellt werden. Es sind zwei verschiedene Arten der Ophthalmoskopie zu unterscheiden.

Bei der so genannten direkten Ophthalmoskopie wird ein Ophthalmoskop sehr nahe zwischen dem zu untersuchenden Auge und dem Betrachterauge gebracht. Die Entfernung liegt bei ca.100 mm zwischen dem Betrachterauge und dem zu untersuchenden Auge, so dass die Untersuchung oft als unangenehm empfunden wird, insbesondere für den Patienten. Bei der direkten Ophthalmoskopie können die zentralen Anteile des Auges, wie Gefäßursprünge oder der Sehnervenkopf, einfach und deutlich vergrößert betrachtet werden.

Bei der so genannten indirekten Ophthalmoskopie wird aus einer Distanz von ca. 150-200 mm mit Hilfe einer Lichtquelle und einem ca. 10-15 mm vor das zu untersuchende Auge gehaltenen optischen Element, insbesondere einer Linse, der Hintergrund des Auges betrachtet. Hierbei lassen sich die Netzhaut (Retina), die Netzhautperipherie, der Sehnerv, die Gefäße sowie der gelbe Fleck leicht untersuchen. Die Vergrößerung ist geringer, als bei der direkten Ophthalmoskopie. Der Überblick über das Auge ist im Gegensatz zur direkten Ophthalmoskopie wesentlich besser und eine stereoskopische (3D) Betrachtung des Auges ist möglich.

Bei der indirekten Ophthalmoskopie kann die Untersuchung des Augenhintergrundes auch mit einer Spaltlampe durchgeführt werden. Bei einer Spaltlampen-Untersuchung kann das Netzhautbild vergrößert werden oder unter Projektion eines Lichtspaltes beurteilt werden. Zur Spaltlampen-Untersuchung wird an einem Mikroskop eine Spaltlampe befestigt. Die Spaltlampe sendet ein schmales, spaltförmiges Lichtbündel aus. Das schmale Lichtbündel der Spaltlampe ermöglicht einen optischen Schnitt durch die transparenten Abschnitte des Augengewebes. So lässt sich die Feinstruktur, Lage und Dicke der Gewebe gut erkennen. Mit der Spaltlampe können die vorderen Augenabschnitte, wie die Hornhaut, die Bindehaut, die Lederhaut, die Regenbogenhaut, die Vorderkammer des Auges und die Linse genau beurteilt werden. Schaltet man bestimmte Zusatzgeräte, wie z.B. eine starke Linse vor das Mikroskop, so lassen sich auch Glaskörper, Netzhaut und Sehnervenkopf untersuchen.

Vorteil der indirekten Ophthalmoskopie gegenüber der direkten Ophthalmoskopie ist die größere Übersicht. Allerdings erfordert die indirekte Technik etwas mehr Übung vom Augenarzt.

Bei Untersuchungen des Augenhintergrundes mit Hilfe der zuvor beschriebenen Verfahren treten aber auch Schwierigkeiten auf. So sind Lichtreflexe bei Augenoperationen sehr störend. Lichtreflexe entstehen an dem vor das zu untersuchende Auge gehaltenen optischen Element, insbesondere einer Linse, oder an der Hornhaut (Cornea) des untersuchten Auges durch die Beleuchtungseinrichtung des Operationsmikroskops oder der Operationssaalbeleuchtung. Diese Lichtreflexe blenden den Betrachter des zu untersuchenden Auges und verschlechtern dadurch das Untersuchungsergebnis.

Aus dem Stand der Technik sind verschiedene Vorrichtungen bekannt, um Lichtreflexe zu reduzieren oder zu vermeiden. So sind aus der DE 3339172 A1 so genannten Lichtfallen für Augenuntersuchungsgeräte bekannt, um die Netzhaut des Auges nicht durch Beleuchtungsstrahlen zu belasten. So ist dort offenbart, dass in einem zentralen Bereich des Beleuchtungsstrahlenganges eines Operationsmikroskops in einer zu Objektebene konjugierten Ebene eine lichtabsorbierende Schicht angeordnet ist. Diese lichtabsorbierende Schicht ist als zentraler Teil einer ringförmigen Blendenöffnung ausgebildet. Die in der DE 3339172 A1 beschriebene lichtabsorbierende Schicht (Lichtfalle) hat den Nachteil, dass die durch die lichtabsorbierende Schicht abgedeckte Stelle des zu untersuchenden Auges nicht ausreichend beleuchtet wird, um detaillierte Untersuchungen dort vorzunehmen. Das Augenuntersuchungsgerät ist ferner nicht flexibel ausgebildet, so dass bei unterschiedlichen zu untersuchenden Augen keine entsprechende Anpassung möglich ist. Eine Anpassung der Lichtfallen an unterschiedliche Hornhäute bzw. an verschieden reflektierte Lichtstrahlen ist, während der Untersuchung, nicht möglich.

Aus der DE 19812050 A1 ist ein Augenmikroskop bekannt, bei dem die Beleuchtung des Auges durch die Verwendung von elektronisch bezüglich ihrer Lichtdurchlässigkeit, Lichtreflektion oder Lichtemission ansteuerbarer, im Auflicht oder Durchlicht beleuchteter oder selbstleuchtender Chipbausteine vereinfacht werden soll. Zwar kann durch diese Chipbausteine ein bestimmter Strahl eines Strahlenbündels geblockt werden, aber auch diese Augenmikroskope sind nicht flexibel einstellbar, wenn sowohl von der Linse, als auch von der Hornhaut des zu untersuchenden Auges Licht von verschiedenen Lichtquellen reflektiert wird.

Die EP 1486159 A1 beschreibt ein Ophthalmoskop zur Untersuchung des Hintergrundes eines Auges mit einer zwischen dem Objektiv des Ophthalmoskops und dem zu untersuchenden Auge angeordneten Linse. Durch die Linse werden zum einen die Beobachtungsstrahlen des Betrachters und zum anderen Beleuchtungsstrahlen einer Lichtquelle geführt. Die Linse weist zwei Oberflächen auf, an denen die Strahlen der Beleuchtung reflektiert werden. Um diese den Betrachter störend beeinflussenden Reflektionen zu unterbinden, sieht das Ophthalmoskop der EP 1486159 A1 vor, dass zum einen die Linse kippbar angeordnet ist, um den Reflex oder die Reflexe von dem Objektiv wegzulenken. Dies hat den Nachteil dass das Ophthalmoskop recht aufwendig gestaltet sein muss, da eine mechanische Vorrichtung vorgesehen sein muss, durch die die Linse gekippt werden kann. Von der Hornhaut des zu betrachtenden Auges reflektiertes Licht gelangt weiterhin in den Beobachtungsstrahlengang des Betrachters und stört somit das Sehempfinden des Betrachters. Die bei einem derartigen Ophthalmoskop verwendeten Linsen sind aufgrund der mechanischen Notwenigkeit der Schwenkvorrichtung für die Linse recht flach ausgebildet und weisen maximal 40 Dioptrien auf, was für viele Anwendungen nicht ausreichend hoch ist.

Ein aus der EP 1486159 A1 bekanntes optisches Gerät zur Untersuchung eines Auges bzw. des Augenhintergrundes weist mindestens ein Okular zur Betrachtung des Auges, mindestens einen verschiedene optische Linsen beinhaltenden Tubus, ein dem Auge zugewandt angeordnetes Objektiv, mindestens eine Beleuchtungsquelle zur Beleuchtung des Auges, eine zwischen dem Objektiv und dem zu untersuchenden Auge angeordnete Linse sowie ein fest in dem optischen Gerät angeordnetes Unterbrechungselement auf.

Ausgehend von dem aus der EP 1486159 A1 bekannten optischen Gerät liegt der vorliegenden Erfindung somit die Aufgabe zugrunde, ein optisches Gerät, insbesondere ein Ophthalmoskop, sowie ein Verfahren zur Untersuchung eines Auges zu schaffen, das die Nachteile des Standes der Technik nicht aufweist. Insbesondere soll das optische Gerät vermeiden, dass Lichtreflektionen an dem optischen Element oder der optischen Anordnung, welche(s) zwischen einem Objektiv des optisches Gerätes und dem zu untersuchenden Auge abgeordnet ist, und an der Hornhaut des zu untersuchenden Auges dem Betrachter störend ins Auge fallen. Ferner soll das optische Gerät derart flexibel einstellbar sein, dass die Lichtreflexionen verschieden gewölbter Hornhäute berücksichtigt werden können.

Die Aufgabe wird erfindungsgemäß durch ein optisches Gerät gemäß Patentanspruch 1, durch ein Verfahren gemäß Patentanspruch 12 sowie die Verwendung eines derartigen erfindungsgemäßen optischen Gerätes gemäß Patentanspruch 15 gelöst. Weitere Ausgestaltungen der Erfindung ergeben sich aus den abhängigen Ansprüchen. Weitere Vorteile, Merkmale und Details der Erfindung ergeben sich aus den Unteransprüchen, der Beschreibung sowie den Zeichnungen. Vorteile, Merkmale und Details, die im Zusammenhang mit dem erfindungsgemäßen optischen Gerät beschrieben sind, gelten dabei selbstverständlich auch im Zusammenhang mit dem erfindungsgemäßen Verfahren sowie der Verwendung des optischen Gerätes, und umgekehrt.

Ein optisches Gerät zur Untersuchung eines Auges, mit mindestens einem dem Auge zugewandt angeordneten Objektiv, mindestens einer Beleuchtungsquelle zur Beleuchtung des Auges, einem bzw. einer zwischen dem Objektiv und dem zu untersuchenden Auge angeordneten optischen Element und/oder optischen Anordnung, welches bzw. welche ein Zwischenbild der untersuchten Ebene im Augeninneren erzeugt, die mit dem Objektiv betrachtet wird, wobei in dem Beobachtungsstrahlengang des optischen Gerätes zumindest ein ortsaufgelöstes Dämpfungselement angeordnet ist, welches in mindestens zwei Transparenzzustände ortsaufgelöst schaltbar ist, stellt ein optisches Gerät, insbesondere ein Ophthalmoskop, zur Betrachtung des Hintergrundes eines Auges dar, welches vermeidet, dass Lichtreflektionen an der Linse und an der Hornhaut des zu untersuchenden Auges dem Betrachter störend ins Auge fallen. Das optische Gerät ist flexibel einstellbar, so dass die Reflexionen verschieden gewölbter Hornhäute berücksichtigt werden können. Als optisches Element bzw. optische Anordnung kann eine Linse, ein Linsensystem oder ein Vergrößerungssystem vorgesehen sein. Im Vergleich zu dem aus dem Stand der Technik bekannten Unterbrechungselement im Beleuchtungsstrahlengang eines optischen Gerätes, ist das erfindungsgemäße Dämpfungselement im Beobachtungsstrahlengang des optischen Gerätes vorgesehen und kann ortsaufgelöst in Abhängigkeit der Lichtintensität der einzelnen Lichtstrahlen im Beobachtungsstrahlengang in unterschiedliche Transparenzzustände geschaltet werden. D.h., die Transparenz des Dämpfungselementes kann gedämpft werden.

Durch die ortsaufgelöste Schaltungsmöglichkeit des Dämpfungselementes in mindestens zwei Transparenzzustände können in dem Beobachtungsstrahlengang bzw. in dem Beobachtungsstrahlenbündel des optischen Gerätes gezielt Lichtreflexe geblockt werden. D.h., Teile der reflektierten Beleuchtungsstrahlen können in dem Beobachtungsstrahlengang des optischen Gerätes ausgeblendet werden. Die Beleuchtung des Auges kann bei einem derartigen optischen Gerät gleichmäßig bleiben, während die Lichtreflexe an dem optischem Element, insbesondere an einer Linse, welche zwischen dem Objektiv des optischen Gerätes und dem zu untersuchenden Auge abgeordnet ist, und die Lichtreflexe an der Hornhaut des zu untersuchenden Auges in dem Beobachtungsstrahlengang geblockt werden können, so dass diese dem Betrachter nicht störend ins Auge fallen. Vorteilhaft ist ferner bei einem derartigen optischen Gerät, dass neben den reflektierten Lichtstrahlen der Beleuchtungseinrichtung auch Lichtstrahlen bzw. reflektierte Lichtstrahlen der Beleuchtung des Operationsraumes geblockt werden können.

Ortsaufgelöst bedeutet, dass über den Querschnitt des Beobachtungskanals einzelne Bereiche des Dämpfungselements unterschiedlich geschaltet werden können. D.h., das Dämpfungselement ist in viele Bereiche unterteilt, die unterschiedlich transparent geschaltet werden können. Der Querschnitt des Dämpfungselements gleicht zum Beispiel einer Matrix mit vielen kleinen Quadraten, die einzeln ansteuerbar sind.

So kann das Dämpfungselement bzw. einzelne Bereiche des Dämpfungselements beispielsweise zwischen einem transparenten und einem diffusen Zustand geschaltet werden. Im diffusen Zustand blockt bzw. verschließt ein Bereich des Dämpfungselements Teile des Strahlenganges, in dem das Dämpfungselement angeordnet ist. Somit übernimmt das Dämpfungselement die Aufgabe von partiellen Verschlussblenden bzw. so genannten Lichtfallen. Allerdings blockt das Dämpfungselement Lichtreflexe an dem optischem Element, insbesondere an einer Linse, oder der Hornhaut des Auges im Beobachtungsstrahlengang und nicht, wie im Stand der Technik bekannt, in den Beleuchtungsstrahlengängen.

Im transparenten Zustand blockt das Dämpfungselement bzw. blocken die jeweiligen ortsaufgelösten Bereiche des Dämpfungselements den/die Beobachtungsstrahlengang/-gänge bzw. Teile des/der Beobachtungsstrahlengangs/gänge des optischen Gerätes nicht, so dass reflektierte Lichtstrahlen ungehindert durch den/die Beobachtungsstrahlengang/-gänge des optischen Gerätes in das Auge des Betrachters gelangen können.

Vorteilhaft ist es, wenn das Dämpfungselement eine so genannte Blockmatrix darstellt. D.h., die Querschnittsfläche des Dämpfungselementes ist in eine Vielzahl von Blöcken unterteilt, wobei jeder einzelne Block zwischen zumindest zwei Transparenzzuständen, insbesondere von einem transparenten Zustand in einen diffusen Zustand, geschaltet werden kann. Das Dämpfungselement kann auch derart ausgebildet sein, dass dieses einen oder mehrere verschwenkbare Zeiger aufweist, wobei das oder die Ende(n) des/der Zeiger(s) die Größe eines oder mehrerer Blockes/Blöcke aufweist/aufweisen. Je nach Bedarf kann der oder können die Zeiger gezielte Blöcke des Dämpfungselementes abdecken und so Teile eines Beobachtungsstrahlenganges blockieren.

Bei dem erfindungsgemäßen optischen Gerät sitzt das Dämpfungselement an der dem Auge abgewandten Seite des optischen Elementes oder der optischen Anordnung, insbesondere an einer Linse. Hierdurch können einfach Lichtreflexionen, welche beispielsweise von den beiden Oberflächen einer Linse ausgehen, unterdrückt werden. Vorteilhafterweise sitzt das Dämpfungselement direkt oberhalb der Linse. Störende Lichtstrahlen, die an beiden Oberflächen der Linse reflektiert werden, können so direkt abgeblockt werden. In diesem Bereich direkt oberhalb der Linse, d.h. an der dem zu betrachtenden Auge abgewandten Seite der Linse, ist die Streuung der reflektierten Lichtstrahlen am kleinsten, so dass nur wenige Bereiche/Blöcke des Dämpfungselementes in einen diffusen Zustand versetzt werden müssen. Die Linse selbst kann dabei direkt auf dem zu untersuchenden Auge befestigt sein.

In einer bevorzugten Ausführungsform des optischen Gerätes ist das zumindest eine Dämpfungselement entlang einer Halterung des optischen Gerätes verschiebbar befestigt. Hierdurch kann das Dämpfungselement je nach Erfordernis einfach in unterschiedliche Positionen in dem Beobachtungsstrahlengang positioniert werden.

Ein zweites Dämpfungselement kann beispielsweise an der Halterung zwischen dem Objektiv und dem ersten Dämpfungselement verschiebbar befestigt sein. Durch das zweite Dämpfungselement können von der Hornhaut des Auges reflektierte Lichtstrahlen abgeblockt werden, so dass auch diese aus dem bzw. den Beobachtungsstrahlenbündel(n) herausgefiltert werden können und der Betrachter des zu untersuchenden Auges eine optimale Sicht auf das Auge hat. Dadurch, dass das zweite Dämpfungselement entlang der Halterung zwischen dem Objektiv und dem ersten Dämpfungselement verschiebbar befestigt ist, kann das erfindungsgemäße optische Gerät leicht auf jede Hornhaut des zu untersuchenden Auges eingestellt werden. D.h., aufgrund der unterschiedlichen Wölbungen der Hornhäute verschiedener Augen werden die auf das jeweilige Auge einfallenden Lichtstrahlen unterschiedlich reflektiert. Durch das verschiebbar gelagerte zweite Dämpfungselement sind die unterschiedlich reflektierten Lichtstrahlen besonders einfach abblockbar. Die zusätzliche Einstellbarkeit der Transparenz der Bereiche des zweiten Dämpfungselementes, insbesondere von einem transparenten Zustand in einen diffusen Zustand und umgekehrt, schafft eine besonders einfache und gute Abblockung der von der Hornhaut reflektierten Lichtstrahlen.

Die Halterung ist vorzugsweise eine Schiene, eine Kulissenführung, ein Stab oder Ähnliches, an der/dem das zumindest eine Dämpfungselement entlang geführt werden kann. Mit Hilfe von Befestigungselementen an der Halterung und entsprechenden Befestigungselementen an dem zumindest einen Dämpfungselement kann dieses entlang der Halterung in jeder gewünschten Position befestigt werden. Die Höhen-Einstellung des zumindest einen Dämpfungselementes kann mechanisch oder elektrisch erfolgen.

Von Vorteil ist ein optisches Gerät, bei dem das zumindest eine Dämpfungselement in seiner Neigung veränderbar an der Halterung befestigt ist. Das Dämpfungselement kann somit orthogonal oder um einen Winkel geneigt in dem Beobachtungsstrahlengang des optischen Gerätes platziert werden.

Besonders bevorzugt ist ferner ein optisches Gerät, bei dem das zumindest eine Dämpfungselement außerhalb der Zwischenbildebene der Netzhaut des zu betrachtenden Auges in dem Beobachtungsstrahlengang des optischen Gerätes angeordnet ist. Außerhalb der Zwischenbildebene der Netzhaut bedeutet, dass das zumindest eine Dämpfungselement in einer gewissen Entfernung zu der Zwischenbildebene der Netzhaut in dem Beobachtungsstrahlengang des optischen Gerätes angeordnet ist. Durch Anordnung außerhalb der Zwischenbildebene der Netzhaut wird das betrachtete Bild der Netzhaut wenig oder nicht beeinflusst. Das zumindest eine Dämpfungselement soll nicht in der Zwischenbildebene des untersuchten Objektes, d.h. des interessanten Bereiches, angeordnet sein, sondern bevorzugt direkt dort, wo Lichtreflexe entstehen und/oder in der Bildebene oder in der Nähe der Bildebene des Entstehungsortes der Lichtreflexe.

Vorteilhaft ist ferner ein optisches Gerät, bei dem das zumindest eine Dämpfungselement in bzw. nahe der Zwischenbildebene der Hornhaut des zu betrachtenden Auges und/oder oberhalb der Zwischenbildebene der Hornhaut des zu betrachtenden Auges in dem Beobachtungsstrahlengang des optischen Gerätes angeordnet ist. Je näher das zumindest eine Dämpfungselement an der Zwischenbildebene der Hornhaut im Beobachtungsstrahlengang angeordnet ist, desto schärfer kann gegen Lichtreflexe an der Hornhaut vorgegangen werden, d.h. desto besser können die störenden Lichtreflexe durch das zumindest eine Dämpfungselement geblockt werden.

Durch die unterschiedlichen Wölbungen der Hornhaut von verschiedenen Augen ist die Zwischenbildebene, d.h. die orthogonal zur optischen Achse des optischen Gerätes liegende Ebene, in der sich das Bild der Hornhaut befindet, in unterschiedlichen Entfernungen zu dem optischem Element, insbesondere zu einer Linse, und zum Objektiv angeordnet. Dadurch, dass das zumindest eine Dämpfungselement verschiebbar an der Halterung gelagert ist, kann dieses leicht in oder nahe an die jeweilige Zwischenbildebene der Hornhaut verschoben werden. Die an der Hornhaut des, mit dem optischen Gerät, betrachteten Auges reflektierten Lichtstrahlen werden dann besonders einfach durch das zumindest eine Dämpfungselement abgeblockt. Die Zwischenbildebene der Hornhaut ist in der Regel deutlich beabstandet zu der Zwischenbildebene der Netzhaut des Auges abgebildet, so dass das zumindest eine Dämpfungselement ausreichend beabstandet von der Zwischenbildebene der Netzhaut in dem Beobachtungsstrahlengang vorgesehen ist. Um die Bildebenen der Hornhaut und der Netzhaut zu trennen wird bevorzugt ein optisches Element oder eine optische Anordnung mit minimaler Schärfentiefe verwendet.

Bevorzugt ist es, wenn ein Dämpfungselement direkt in der Zwischenbildebene oder etwas oberhalb der Zwischenbildebene der Hornhaut einer Ophthalmoskopierlupe eines Ophthalmoskops sitzt. Hierdurch können Lichtreflexe an der Ophthalmoskopierlupe besonders effektiv geblockt werden.

Ein optisches Gerät, bei dem die Linse an der Halterung lösbar befestigt ist, stellt ein sehr flexibles optisches Gerät dar, da unterschiedliche Linsen mit unterschiedlichen Stärken auf diese Art und Weise an dem optischen Gerät befestigt werden können. Um sehr detaillierte Ansichten beispielsweise des Augenhintergrundes zu erhalten, können Linsen mit Dioptrienzahlen von über 40 Dioptrien verwendet werden. Soll nur eine grobe Untersuchung des Auges stattfinden, können Linsen mit Dioptrienzahlen von weniger als 40 Dioptrien an der Halterung befestigt werden.

Vorteilhaft ist ferner ein optisches Gerät, bei dem das optische Element oder die optische Anordnung in ihrer Neigung verstellbar ist. Durch die Neigungsmöglichkeit des optischen Elementes, insbesondere einer Linse, an der Halterung können die an den beiden Oberflächen der Linse reflektierten Lichtstrahlen in einem Punkt zusammengeführt werden. Die Zusammenführung erfolgt bevorzugt in der Ebene in der zumindest ein Dämpfungselement angeordnet ist. Hierdurch können die störenden, an der Linse reflektierten Lichtstrahlen besonders einfach abgeblockt werden. Die Neigung des optischen Elementes oder der optischen Anordnung kann durch mechanische oder elektrische Verstellung erfolgen.

In einer weiteren bevorzugten Ausführungsform des optischen Gerätes ist eine Beleuchtungsquelle an der Halterung verschiebbar gelagert. Durch die verschiebbare Befestigung der Beleuchtungsquelle an der Halterung können leicht unterschiedliche Beleuchtungen des Auges realisiert werden. So kann das Beleuchtungsstrahlenbündel zylinderförmig, konisch oder gekreuzt auf die Linse des optischen Gerätes geführt werden.

Das zumindest eine Dämpfungselement stellt vorzugsweise einen elektrooptischen Schalter dar, welcher elektronisch angesteuert werden kann. Durch die elektronische Ansteuerung des zumindest einen Dämpfungselementes kann ein schnelles Umschalten der jeweiligen Bereiche des zumindest einen Dämpfungselementes zwischen den unterschiedlichen Transparenzzuständen gewährleistet werden. Neben dem Zustand des vollständigen Blockierens der Strahlen, d.h. dem diffusen Zustand, oder des vollständigen Durchlassens von Strahlen, d.h. dem transparenten Zustand, kann auch jeder Transparenzzustand zwischen beiden Extremen realisiert werden. Dies ist durch den Einsatz einer Blende, wie sie aus dem Stand der Technik bekannt ist, nicht möglich. Auch zeitliche Verläufe können hierbei voreingestellt werden, wodurch ein zeitliches Zustandsmuster des zumindest einen Dämpfungselementes bzw. einzelner Bereiche des zumindest einen Dämpfungselementes realisiert werden kann.

Das zumindest eine Dämpfungselement ist vorzugsweise als ein Flüssigkristall-Element, eine Polymer-Verschlussblende, eine LCD-Matrix oder eine Elektrowetting-Matrix ausgebildet. Eine elektronisch schaltbares Flüssigkristall-Element, eine Polymer-Verschlussblende, eine LCD-Matrix oder eine Elektrowetting-Matrix sind besonders vorteilhaft, da diese zum einen zuverlässig in zwei verschiedene Transparenzzustände gebracht werden können und andererseits eine sehr hohe Reaktionsgeschwindigkeit bei der Ansteuerung besitzen. Als Polymer-Shutter wird insbesondere ein optisches Element bezeichnet, das auf der Basis elektronisch steuerbarer Lichtstreuung arbeitet. Dieses optische Element wird von einem externen elektrischen Feld gesteuert, wobei das optische Element durch die entsprechende Ausrichtung der Kristalle hoch transparent ist, wenn das elektrische Feld abgeschaltet ist, und dem optischen Element durch Anlegen des elektrischen Feldes ein hoher Trübungsgrad und damit ein hohes Streuungsvermögen verliehen wird. Polymer-Shutter arbeiten mit unpolarisiertem Licht und ermöglichen über den gesamten sichtbaren Bereich eine hohe Transmission. Vorteilhaft sind Polymer-Shutter einsetzbar, die eine Reaktionszeit im Submillisekunden-Bereich aufweisen.

Die vorliegende Erfindung ist nicht auf eine bestimmte Ausführungsform eines Polymer-Shutters beschränkt. Eine mögliche Ausführungsform kann beispielsweise durch ein Paar Glasscheiben mit einer dazwischen angeordneten aktiven Schicht gebildet sein, wobei die aktive Schicht freie Flüssigkristall-Moleküle aufweist. Diese können durch eine Photopolymerisation von Flüssigkristall-Polymermolekülen in Gegenwart von herkömmlichen Flüssigkristallen erhalten werden.

Bei dem Polymer-Shutter können beispielsweise transparente Elektroden zum Aufbringen des elektrischen Feldes verwendet werden.

Die Spannung, mit der der Polymer-Shutter beaufschlagt werden kann, kann beispielsweise bei 200V liegen, wobei dies die Differenz der Maxima eines Spannungsverlaufs darstellt. Zum Betrieb eines derartigen oder mehrerer derartiger Polymer-Shutter(s) müssen lediglich zusätzliche elektrische Anschlüsse an dem/den Polymer-Shutter(n) vorgesehen sein.

Unter Ansteuern beziehungsweise Aktivieren des zumindest einen Dämpfungselementes wird im Sinne der Erfindung das Versetzen des zumindest einen Dämpfungselementes bzw. der einzelnen Bereiche des zumindest einen Dämpfungselementes in einen anderen Transparenzzustand, insbesondere in den diffusen Zustand, verstanden. Bei einem Dämpfungselement, das auf elektronischer Basis arbeitet, bedeutet somit Ansteuern das Anlegen einer erforderlichen Spannung zur Einstellung des Transparenzzustandes.

Bevorzugt weist das optische Gerät eine Betätigungsvorrichtung zur Ansteuerung des zumindest eine Dämpfungselementes auf. Diese Betätigungsvorrichtung kann beispielsweise ein Schalter sein, über den das zumindest eine Dämpfungselement oder Bereiche des zumindest einen Dämpfungselementes aktiviert und deaktiviert werden kann bzw. können. Hierbei ist vorzugsweise jedem Dämpfungselement ein eigener Schalter zugeordnet, um ein separates Ansteuern jedes Dämpfungselementes ermöglichen zu können.

Bevorzugt ist ein optisches Gerät, bei dem die Linse eine Stärke von mehr als 40 Dioptrien aufweist. Dies ist für detaillierte Betrachtungen des Auges von Vorteil.

Die Aufgabe der Erfindung wird ferner durch ein Verfahren zum Blocken von Lichtreflexen im Beobachtungsstrahlengang eines optischen Gerätes, welches zur Untersuchung eines Auges, insbesondere der Netzhaut eines Auges, ausgebildet ist, bei dem das Blocken der Lichtreflexe im Beobachtungsstrahlengang mit einen zuvor erwähnten erfindungsgemäßen optischen Gerät erfolgt, gelöst. Dadurch, dass einzelne Bereiche/Blöcke des Dämpfungselementes in zumindest zwei unterschiedliche Transparenzzustände versetzt werden können, können Lichtreflexe von dem optischen Element, insbesondere von einer Linse, sowie von der Hornhaut des untersuchten Auges gezielt abgeblockt werden. Da, wo die Lichtreflexe in dem Strahlenbündel des Beobachtungsstrahlengangs verlaufen, werden die Bereiche des zumindest einen Dämpfungselementes verdunkelt, so dass die Lichtreflexe nicht mehr zu dem Auge des Betrachters gelangen können. D.h., die Bereiche/Blöcke des Dämpfungselementes werden in Abhängigkeit von der Lichtintensität der einzelnen Beobachtungsstrahlen des Beobachtungsstrahlengangs geschaltet. Bei Überschreitung einer zuvor festgelegten Lichtintensität wird die Transparenz der jeweiligen Bereiche/Blöcke des Dämpfungselementes verändert. Bei hoher Lichtintensität werden die Bereiche/Blöcke des Dämpfungselementes diffus geschaltet, so dass die hellen reflektierten Lichtstrahlen geblockt werden. Bei geringer bzw. normaler Lichtintensität bleiben alle Bereiche/Blöcke des Dämpfungselementes transparent geschaltet. Das kann manuell aber auch automatisch geschehen, in dem das beobachtete Bild ausgekoppelt, mit z.B. einer Kamera aufgenommen und von einer Software ausgewertet wird und eine Regelung zur Unterdrückung der Reflexe gestartet wird. Eine solche Anordnung trägt den unvermeidlichen Augen- und / oder Kopfbewegungen des Patienten Rechnung.

Bevorzugt ist ein Verfahren zum Blocken von Lichtreflexen im Beobachtungsstrahlengang eines optischen Gerätes, bei dem das optische Gerät, insbesondere das Objektiv des optischen Gerätes, mit einer geringen Schärfentiefe betrieben wird. Hierdurch können Zwischenbilder bzw. Zwischenbildebenen im Beobachtungsstrahlengang leicht getrennt werden.

Die Verwendung eines zuvor erwähnten erfindungsgemäßen optischen Gerätes zur Untersuchung eines Auges, insbesondere der Netzhaut des Auges, ermöglicht dem Betrachter bzw. dem Operateur eine optimale Betrachtung des zu untersuchende Auges. Durch die Verwendung zumindest eines ortsaufgelösten Dämpfungselementes können gezielt störende Lichtreflexe, die von dem optischen Element, insbesondere der Linse, des optischen Gerätes sowie von dem zu untersuchenden Auges, insbesondere der Hornhaut des Auges, ausgehen, geblockt werden.

Das erfindungsgemäße optische Gerät stellt ein Beobachtungsgerät, insbesondere ein Ophthalmoskop oder ein Mikroskop, insbesondere mit einer Spaltlampe, dar. Das optische Element ist bevorzugt eine Linse hoher Brechkraft.

Die Erfindung wird nachfolgend anhand von Ausführungsbeispielen unter Bezugnahme auf die beiliegenden Zeichnungen näher erläutert. Es zeigen:
- Figur 1: eine schematische Darstellung der von der Hornhaut und der Netzhaut des zu untersuchenden Auges reflektierten Strahlengänge;
- Figur 2: schematisch eine Ausführungsform der Halterung mit Dämpfungselement und optischem Element eines erfindungsgemäßen optischen Gerätes;
- Figur 3: schematisch eine weitere Ausführungsform der Halterung, des zumindest einen Dämpfungselementes und des optischen Elementes des erfindungsgemäßen optischen Gerätes;
- Figur 4: ein Dämpfungselement mit geblockten Bereichen;
- Figur 5: ein Dämpfungselement mit Zeiger.

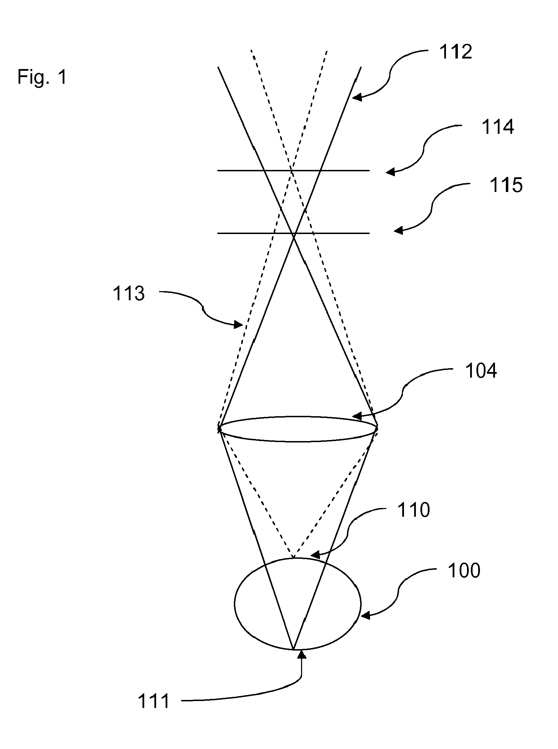

Fig. 1 zeigt eine schematische Darstellung der von der Hornhaut 110 und der Netzhaut 111 des zu untersuchenden Auges 100 reflektierten Strahlengänge 113 bzw. 112. Die Zwischenbildebene 114 der Hornhaut 110 liegt zwischen dem optischen Element 104, hier einer Linse, und dem nicht dargestellten Objektiv. Die Zwischenbildebene 115 der Netzhaut 111 liegt zwischen der Zwischenbildebene 114 der Hornhaut 110 und der Linse 104. Um die Zwischenbildebene 115 der Netzhaut 111 und die Zwischenbildebene 114 der Hornhaut 110 zu trennen, wird bevorzugt ein optisches Gerät, insbesondere ein Operationsmikroskop bzw. ein Ophthalmoskop, mit geringer Schärfentiefe betrieben. Durch die Beleuchtung des Auges 100 durch an dem optischen Gerät angeordneten Beleuchtungsquellen, werden an der Hornhaut 110 Lichtstrahlen reflektiert, die den Betrachter bei der Untersuchung des Auges 100 störend beeinflussen. Diese Lichtreflexe gilt es zu blocken, so dass ein verbessertes Untersuchungsergebnis erhalten werden kann.

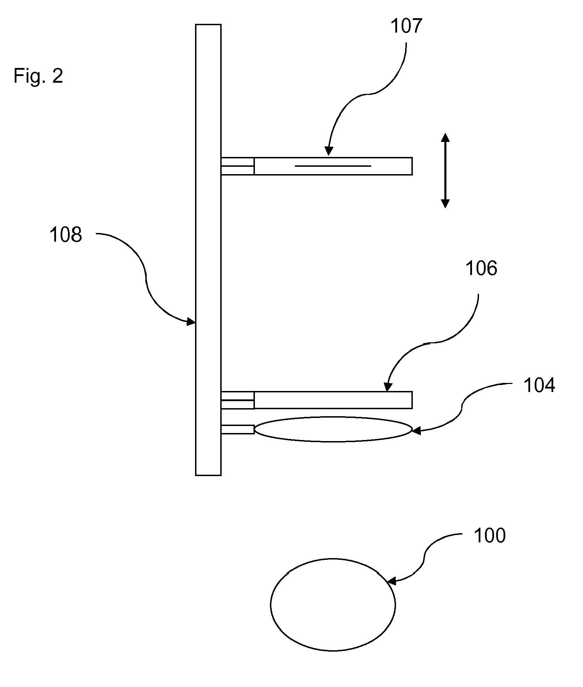

In der Fig. 2 ist schematisch eine Ausführungsform der Halterung 108, zweier Dämpfungselemente 106, 107 und eines optischen Elementes 104, hier einer Linse, eines erfindungsgemäßen optischen Gerätes dargestellt. Die Halterung 108 sitzt am unteren Ende des optischen Gerätes dem Auge 100 zugewandt. Die Linse 104 ist an dem dem Auge 100 zugewandten Ende der Halterung 108 angeordnet, so dass die Linse 104 bei der Untersuchung des Auges 100 bevorzugt in einer Entfernung von ungefähr 50mm bis 150mm vor dem Auge sitzt. Das nicht dargestellte Objektiv des optischen Gerätes sitzt bevorzugt in einer Entfernung von 400mm bis 600mm von dem Auge 100. An der dem Auge 100 abgewandten Seite der Linse 104 ist ein erstes Dämpfungselement 106 an der Halterung 108 angeordnet. Vorzugsweise sitzt das erste Dämpfungselement 106 direkt vor der Linse 104, um so direkt die an den Oberflächen der Linse 104 reflektierten störenden Lichtstrahlen zu blocken. Das zweite Dämpfungselement 107 sitzt in einer größeren Distanz, als das erste Dämpfungselement 106, von der Linse 104 entfernt. Das zweite Dämpfungselement 107 blockt vorzugsweise die von der Hornhaut 110 des Auges 100 reflektierten störenden Lichtstrahlen. Um ein optimales Blockieren der reflektierten Lichtstrahlen zu gewährleisten, ist das zweite Dämpfungselement 107 entlang der Halterung 108 verschiebbar befestigt. Hierdurch kann das zweite Dämpfungselement 107 genau in die Zwischenbildebene 114 der Hornhaut 110 eines jeden Auges 100 platziert werden. Da die Hornhaut 110 bei jedem Auge 100 verschieden ist, ist so eine optimale Anpassung an jedes Auge 100 gewährleistet.
Einzelne oder mehrere Bereiche des ersten Dämpfungselement 106 und des zweiten Dämpfungselement 107 sind dabei zwischen zumindest zwei Transparenzzuständen, insbesondere einem hoch transparenten Zustand und einem Streuzustand, schaltbar. D.h., in dem Streuzustand, auch diffuser Zustand genannt, blocken die entsprechenden Bereiche die reflektierten Lichtstrahlen. Das erste Dämpfungselement 106 und das zweite Dämpfungselement 107 übernehmen die Aufgabe von partiellen Verschlussblenden bzw. von Lichtfallen. In dem transparenten Zustand behindern die Dämpfungselemente 106, 107 bzw. die Bereiche der Dämpfungselemente 106, 107 den/die Beobachtungsstrahlengang/-gänge nicht. Die beiden Dämpfungselemente 106, 107 stellen so genannte Blockmatrixen dar. D.h., die Querschnittsfläche der Dämpfungselemente 106, 107 sind in eine Vielzahl von Blöcken unterteilt, wobei jeder einzelne Block in zumindest zwei Transparenzzustände, insbesondere in einen volltransparenten Zustand und in einen diffusen Zustand, schaltbar ist.

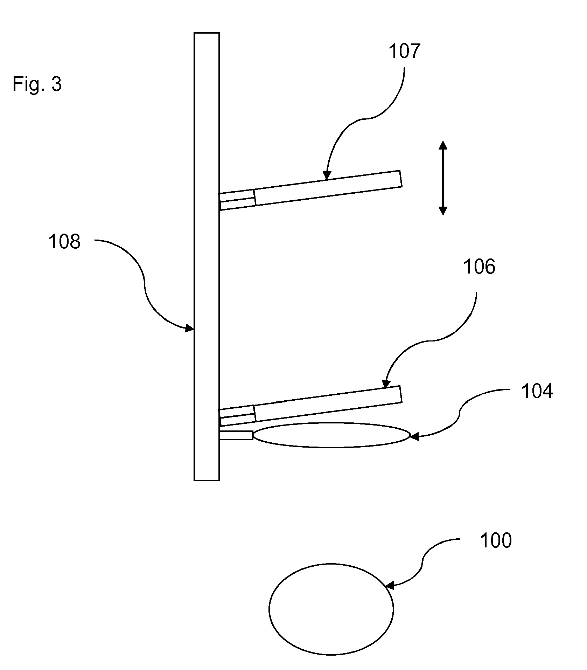

Fig. 3 zeigt ein weiteres Ausführungsbeispiel einer Halterung 108 mit Dämpfungselementen 106, 107 und einer Linse 104 des erfindungsgemäßen optischen Gerätes. Im Gegensatz zu Fig. 2 sind die beiden Dämpfungselemente 106, 107 geneigt angeordnet. D.h., die beiden Dämpfungselemente 106, 107 sind in ihrer Neigung variierbar. Die Dämpfungselemente 106, 107 können derart geneigt werden, dass sie selber keinen Beitrag zu einer störenden Lichtreflexion leisten.

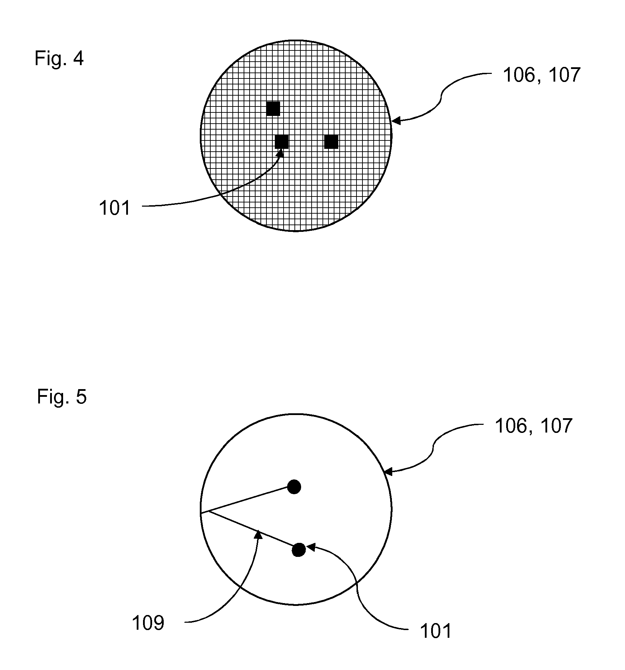

In den Fig. 4 und 5 ist jeweils ein Querschnitt durch ein Dämpfungselement 106, 107 dargestellt. In Fig. 4 sind die diffusen Bereiche 101 der Dämpfungselemente 106, 107 schwarz dargestellt. Die Dämpfungselemente 106, 107 sind in eine Vielzahl von Bereichen/Blöcken unterteilt. D.h., jedes Dämpfungselement 106, 107 stellt eine so genannte Blockmatrix dar. Jeder einzelne Block eines jeden Dämpfungselementes 106, 107 ist in zumindest zwei verschiedene Transparenzzustände, insbesondere von einem transparenten Zustand in einen diffusen Zustand, schaltbar. Je nachdem wo die störenden Lichtreflexe in dem Beobachtungsstrahlengang des optischen Gerätes verlaufen, können die Bereiche/Blöcke so geschaltet werden, dass genau diese störenden Lichtreflexe abgeblockt werden.
Das Dämpfungselement 106, 107 kann auch derart ausgebildet sein, dass dieses einen oder mehrere verschwenkbare Zeiger 109 aufweist, wobei das oder die Ende(n) des/der Zeiger(s) die Größe eines oder mehrerer Raster(s) aufweist/aufweisen, siehe Fig. 5. Je nach Bedarf kann der oder können die Zeiger 109 gezielte Bereiche eines Dämpfungselementes 106, 107 abdecken und so Teile eines Beobachtungsstrahlenganges blockieren.

### Bezugszeichenliste

- 100: untersuchtes Auge
- 101: diffuse Bereiche eines Dämpfungselementes
- 104: optisches Element (Linse)
- 106: erstes Dämpfungselement
- 107: zweites Dämpfungselement
- 108: Halterung
- 109: Zeiger eines Dämpfungselementes
- 110: Hornhaut
- 111 1: Netzhaut
- 112: Strahlengang der Netzhaut
- 113: Strahlengang der Hornhaut
- 114: Zwischenbildebene der Hornhaut
- 115: Zwischenbildebene der Netzhaut

## Patentansprüche

1. Optisches Gerät zur Untersuchung eines Auges, mit mindestens einem dem Auge zugewandt angeordneten Objektiv, mindestens einer Beleuchtungsquelle zur Beleuchtung des Auges, einem bzw. einer zwischen dem Objektiv und dem zu untersuchenden Auge angeordneten optischen Element und/oder optischen Anordnung (104), welches bzw. welche ein Zwischenbild der untersuchten Ebene im Augeninneren erzeugt, die mit dem Objektiv betrachtet wird, **dadurch gekennzeichnet, dass** in dem Beobachtungsstrahlengang des optischen Gerätes zumindest ein ortsaufgelöstes Dämpfungselement (107) angeordnet ist, welches in mindestens zwei Transparenzzustände ortsaufgelöst schaltbar ist.

2. Optisches Gerät gemäß Anspruch 1, **dadurch gekennzeichnet, dass** das zumindest eine Dämpfungselement (107) entlang einer Halterung (108) des optischen Gerätes verschiebbar befestigt ist und/oder dass das zumindest eine Dämpfungselement (107) in seiner Neigung veränderbar an der Halterung (108) befestigt ist.

3. Optisches Gerät gemäß einem der Ansprüche 1 bis 2, **dadurch gekennzeichnet, dass** das zumindest eine Dämpfungselement (107) außerhalb der Zwischenbildebene der Netzhaut des zu betrachtenden Auges in dem Beobachtungsstrahlengang des optischen Gerätes angeordnet ist.

4. Optisches Gerät gemäß einem der Ansprüche 1 bis 3, **dadurch gekennzeichnet, dass** das zumindest eine Dämpfungselement (107) in bzw. nahe der Zwischenbildebene der Hornhaut des zu betrachtenden Auges und/oder oberhalb der Zwischenbildebene der Hornhaut des zu betrachtenden Auges in dem Beobachtungsstrahlengang des optischen Gerätes angeordnet ist.

5. Optisches Gerät gemäß einem der Ansprüche 1 bis 4, **dadurch gekennzeichnet, dass** das zumindest eine Dämpfungselement (107) nahe an der Lupe auf der objekt- bzw. augenabgewandten Seite angeordnet ist.

6. Optisches Gerät gemäß einem der Ansprüche 1 bis 5, **dadurch gekennzeichnet, dass** das zumindest eine Dämpfungselement (107) spektralaufgelöst ausgebildet ist.

7. Optisches Gerät gemäß einem der Ansprüche 1 bis 6, **dadurch gekennzeichnet, dass** das optischen Element und/oder die optischen Anordnung (104) in ihrer Neigung verstellbar ist.

8. Optisches Gerät gemäß einem der Ansprüche 1 bis 7, **dadurch gekennzeichnet, dass** die Beleuchtungsquelle an der Halterung (108) verschiebbar gelagert ist.

9. Optisches Gerät gemäß einem der Ansprüche 1 bis 9, **dadurch gekennzeichnet, dass** das zumindest eine Dämpfungselement (107) ein elektrooptischer Schalter ist oder dass das zumindest eine Dämpfungselement (107) als ein Flüssigkristall-Element, eine Polymer-Verschlussblende, eine LCD-Matrix oder eine Elektrowetting-Matrix ausgebildet ist.

10. Optisches Gerät gemäß einem der Ansprüche 1 bis 9, **dadurch gekennzeichnet, dass** zumindest zwei Dämpfungselemente (107) separat ansteuerbar sind.

11. Optisches Gerät gemäß einem der Ansprüche 1 bis 10, **dadurch gekennzeichnet, dass** das optische Gerät eine Betätigungsvorrichtung zur Ansteuerung des zumindest einen Dämpfungselements (107) aufweist.

12. Optisches Gerät gemäß einem der Ansprüche 1 bis 11, **dadurch gekennzeichnet, dass** das optische Element und/oder die optische Anordnung (104) eine Stärke von größer 40 Dioptrien aufweist.

13. Verfahren zum Blocken von Lichtreflexen im Beobachtungsstrahlengang eines optischen Gerätes, welches zur Untersuchung eines Auges, insbesondere der Netzhaut eines Auges, ausgebildet ist, **dadurch gekennzeichnet, dass** das Blocken der Lichtreflexe im Beobachtungsstrahlengang mit einen optisches Gerät gemäß einem der Ansprüche 1 bis 12 erfolgt.

14. Verfahren zum Blocken von Lichtreflexen im Beobachtungsstrahlengang eines optischen Gerätes nach Anspruch 13, **dadurch gekennzeichnet, dass** das optische Gerät, insbesondere das Objektiv des optischen Gerätes, mit einer geringen Schärfentiefe betrieben wird.

15. Verwendung eines optischen Gerätes gemäß einem der Ansprüche 1 bis 12 zur Untersuchung eines Auges, insbesondere der Netzhaut des Auges.
